# EUROPÄISCHE PATENTANMELDUNG

(11) **EP 0 595 161 A1**
(43) Veröffentlichungstag der Anmeldung: **04.05.1994**
(21) Anmeldenummer: 93116842.1
(22) Anmeldetag: 19.10.1993
(51) Int. Cl.: A61F 13/14

(54) **Fertigbandage**

(30) Priorität: 30.10.1992 DE 4236654
(71) Anmelder: MIRO KLINIK-UND ÄRZTEBEDARF GmbH, D-51674 Wiehl (DE)
(72) Erfinder: Rothmann, Michael, D-51674 Wiehl (DE)
(74) Vertreter: Stachow, E.-W., Prof. Dr.

(57) **Zusammenfassung**

Die Erfindung betrifft eine Fertigbandage insbesondere zur Behandlung von Schlüsselbeinverletzungen, die im wesentlichen aus zwei in ihrer Länge einstellbaren Bändern aus einem nicht dehnbaren Material besteht, wobei die Bänder jeweils an einer ihrer Schmalseiten flach liegend miteinander verbunden und in einem festen Winkel zueinander angeordnet sind und im Bereich der Verbindung beider Bänder mindestens ein flaches Verbindungselement befestigt ist. Die Bandage wird dem Patienten derart angelegt, daß die Bänder jeweils von der Schulter des Patienten direkt zum Rücken und von der Schulter durch die Achselhöhlen des Patienten zum Rücken verlaufen und dort miteinander verbunden sind.

Durch die Erfindung soll eine Fertigbandage zur Behandlung von Schlüsselbeinverletzungen geschaffen werden, die gute Trageigenschaften aufweist, sowie kostengünstig und leicht anlegbar ist.

Die Erfindung zeichnet sich dadurch aus, daß die Bänder (1, 2) über die gesamte Länge und Breite dreischichtig ausgebildet sind, derart, daß die dem Körper des Patienten zugewandte Unterseite (6) aus einer naturfasernen Textilgewebeschicht besteht, auf der eine dünne Polsterschicht (8) aus einem Schaumstoffmaterial und darauf als Oberseite (7) eine Schicht aus einem Polyamidgewebe angeordnet ist und die drei Schichten (6, 7, 8) über ihre gesamte Länge miteinander fest verbunden sind, daß an der Unterseite (6) im Bereich der direkten Verbindung beider Bänder (1, 2) miteinander ein Rückenpolster (3, 3') angeordnet ist und daß die Bänder (1, 2) lediglich im Bereich der Achselhöhlen mit einer zusätzlichen Unterpolsterung (10) versehen sind.

## Beschreibung

Die Erfindung betrifft eine Fertigbandage insbesondere zur Behandlung von Schlüsselbeinverletzungen, die im wesentlichen aus zwei in ihrer Länge einstellbaren Bändern aus einem nicht dehnbaren Material besteht, wobei die Bänder jeweils an einer ihrer Schmalseiten flach liegend miteinander verbunden und in einem festen Winkel zueinander angeordnet sind und im Bereich der Verbindung beider Bänder mindestens ein flaches Verbindungselement befestigt ist. Dabei wird die Bandage dem Patienten derart angelegt, daß die Bänder jeweils von der Schulter des Patienten direkt zum Rücken und von der Schulter durch die Achselhöhlen des Patienten zum Rücken verlaufen und dort miteinander verbunden sind.

Solcherart Bandagen sind in Fachkreisen als sogenannte Clavicula-Bandagen bekannt und dienen dazu, Schlüsselbeinverletzungen zu behandeln. In der Regel treten diese Verletzungen als Frakturen auf, die als Schräg- oder Splitterbrüche entstehen. Durch Ruhigstellung mit einem sogenannten "Rucksackverband" wachsen die Bruchenden zusammen, ohne aneinander zu reiben.

Üblicherweise besteht ein solcher Rucksackverband aus einem mit Watte gefüllten Trikotschlauch, der von den behandelnden Ärzten beziehungsweise dem Pflegepersonal jeweils direkt am Patienten durch Umschlingen und anschließendes Verknoten der Enden hergestellt wird.

Nachteilig bei diesen an sich kostengünstigen Verbänden ist, daß sich das Material des Verbandes unter Zug ausdehnen kann und demzufolge der Verband regelmäßig nachgespannt werden muß. Für die Dauer der Heilung sind deshalb mehrere Verbände nötig, deren Herstellung äußerst zeitintensiv ist. Außerdem verursacht der Verband beim Patienten Unbehagen, da die Verknotung auf den Rücken drückt und dadurch Druckstellen hervorruft.

Um diese Nachteile zu vermeiden sind eine Reihe Fertigbandagen bekannt geworden, die jedoch Nachteile aufweisen. So sind sie zum Teil aufwendig und kompliziert anzulegen, weisen Verschlußschnallen aus Metall auf und sind aus einem hautunfreundlichen Material hergestellt. Dies birgt die Gefahr des Auftretens von Allergien in sich. Außerdem kann die Haut unter den bandagierten Stellen leicht schwitzen, wodurch ein Juckreiz entstehen kann und der Patient sich unwohl fühlt.

Aus dem deutschen Gebrauchsmuster G 89 00 721.2 ist eine Clavicula-Bandage bekannt geworden, die aus zwei in ihrer Länge einstellbaren Bändern besteht. Die Bänder sind aus einem nicht dehnbaren Material gefertigt und mit je einem ihrer Enden an einem Ring befestigt. Damit sind beide Bänder über diesen Ring miteinander verbunden. Die freien Enden der Bänder weisen Mittel zur Schlaufenbildung auf, um nach Anlegen der Bandage die Bandenden mit Hilfe von Klettverschlüssen entsprechend der gewünschten Länge am Ring befestigen zu können. Die Bandage wird dem Patienten dabei in der eingangs beschriebenen Art angelegt, wobei sowohl der Ring, als auch die um den Ring gelegten Schlaufen unangenehme Druckstellen verursachen können. Außerdem können die Bänder infolge ihrer losen Verbindung mit dem Ring leicht verrutschen, was zu einem mangelhaften Sitz der Bandage führen kann. Die Bänder der Bandage bestehen aus einem doppellagigen Schlingengewebe aus Polyamid mit einer Zwischenlage aus dickem Schaumstoff, die durch mehrere längsgeführte Steppnähte fixiert ist.

Nachteilig an dieser Lösung ist aber, daß die Bandage infolge der starken Unterpolsterung zwar keine Druckstellen verursachen kann, aber unter der Kleidung stark aufträgt. Damit ist die Bandage für jeden Außenstehenden erkennbar. Das eingesetzte Polyamidmaterial gewährleistet zwar einen sicheren und festen Sitz der Bandage, ist jedoch hautunfreundlich und reizt die bandagierten Hautpartien. Damit ist die Gefahr des Auftretens von Allergien und von Juckreiz nicht ausgeschlossen. Außerdem kann auch der Plastering Hautreizungen verursachen, die ebenfalls dazu führen, daß der Patient sich nicht wohl fühlt.

Weitere Schlüsselbeinbandagen offenbaren die US-Patentschriften 37 18 137 und 38 97 776. Bei der US-PS 37 18 137 bestehen die Bänder der Bandage aus einem elastischen Schaumstoffkern, der von einem baumwollnen Gewebe überzogen ist. Dabei ist ein unelastisches Band zwischen Schaumstoffkern und Hülle eingenäht, dessen Enden über den Kern und die Hülle hinausragen und als Verschlußenden mit den schnallenförmigen Verbindungselementen dienen. Der dicke Schaumstoffkern verhindert zwar, daß auf der Haut des Patienten Druckstellen im Bereich der Bänder entstehen können, im direkten Verbindungsbereich der oberen Bandenden ist aber ein solcher Schutz nicht vorhanden. Außerdem trägt die Bandage auf dem Körper des Patienten auf, so daß die Bandage für jedermann sichtbar bleibt.

Aufbau und Herstellung dieser Schlüsselbeinbandage sind ebenso aufwendig wie die nach der US-PS 38 97 776 beschriebene Bandage. Mit dieser Bandage soll der Nachteil des Auftretens von Druckstellen auf der Haut des Patienten dadurch verhindert werden, daß in den gefährdeten Bereichen die Bänder besonders dick unterpolstert sind. Dazu weist ein Kern aus einem Schaumstoffmaterial eine zum Körper zeigende halbrunde Form auf, wobei der Querschnitt des Kerns vom unteren Bandende aus in Richtung Achselhöhle zunehmend dicker wird. Der Kern ist von einem Baumwollgewebe überzogen und die dem Körper abgewandte Bandoberseite enthält zwischen Kern und Baumwollschlauch ein unelastisches Stoffband, dessen Enden über die des Kerns hinausragen und als Verschlußenden für die Schnallen dienen.

Nachteilig an dieser Ausführungsform ist auch, daß weder die Bandenden, die mit den Schnallen verbunden werden, noch die Schnallen selbst unterpolstert sind, so daß hier Druckstellen und damit Hautreizungen entstehen können. Gleiches kann in den Bereichen, in denen die gepolsterten Bänder miteinander bzw. mit den ungepolsterten Bandenden vernäht sind, auftreten.

Schließlich ist mit der US-PS 38 56 004 eine Schlüsselbeinbandage bekannt geworden, die aus zweischichtigen Bändern ausgeführt ist, wobei die Haut des Patienten direkt mit der ersten Schicht, einer Schaumstoffschicht, in Berührung kommt. Die Oberschicht besteht dabei aus einem solchen nicht dehnbaren synthetischen Material, das mit Klettverschlußelementen zusammenwirken kann. Die Befestigung der Bandenden nach dem Anlegen der Bandage geschieht mittels eines dreieckigen Verbindungselementes, durch das die Enden der Bandage geführt und mittels Klettverschluß auf der Bandoberseite befestigt werden. Nachteilig an dieser Lösung ist, daß die Bandenden im Schulterbereich nicht unter einem vorgegebenen festen Winkel miteinander verbunden sind, so daß ein fester, dem Körper angepaßter Sitz der Bandage nicht gewährleistet werden kann. Beim Anlegen der Bandage entstehen im Rückenbereich Falten infolge der winkligen Auseinanderspreizung der beiden Bänder, was zu Druckstellen und Reizungen der Haut führen kann. Bereits vorher beschriebene Nachteile, wie die Erkennbarkeit der Bandage für jedermann, Hautreizungen durch das eingesetzte Material für die Bandage usw. werden mit dieser Lösung auch nicht verhindert.

Es ist deshalb Aufgabe der Erfindung, eine Fertigbandage zur Behandlung von Schlüsselbeinverletzungen zu entwickeln, die gute Trageeigenschaften aufweist, kostengünstig und leicht anlegbar ist und die beschriebenen Nachteile der bereits bekannten Lösungen weitestgehend verhindert.

Erfindungsgemäß wird die Aufgabe dadurch gelöst, daß die Bänder über die gesamte Länge und Breite dreischichtig ausgebildet sind, derart, daß die dem Körper des Patienten zugewandte Unterseite aus einer naturfasernen Textilgewebeschicht besteht. Auf dieser Schicht ist eine dünne Polsterschicht aus einen Schaumstoffmaterial und darauf als Oberseite eine Schicht aus einem Polyamidgewebe angeordnet. Die drei Schichten sind über ihre gesamte Länge fest miteinander verbunden. Weiterhin ist an der Unterseite im Bereich der direkten Verbindung beider Bänder miteinander ein Rückenpolster angeordnet, und die Bänder sind lediglich im Bereich der Achselhöhlen mit einer zusätzlichen Unterpolsterung versehen.

Durch die erfindungsgemäße Lösung wird eine Fertigbandage geschaffen, die besonders einfach zu handhaben ist, einen guten Sitz gewährleistet und die ggf. auf einfache Weise nachgestellt werden kann. Die dreischichtige Ausbildung der Bänder gewährleistet, daß die Haut des Patienten nur mit hautfreundlichen Materialien in Berührung kommt. Dadurch, daß die Oberseite aus einem Polyamidgewebe hergestellt ist, wird erreicht, daß die Fertigbandage nahezu undehnbar ist und einen andauernden straffen Sitz sichert. Die großflächige Unterlegung der Verbindungsstelle der Bandage im Rücken des Patienten mit einem Rückenpolster sorgt dafür, daß der Patient nicht durch Druckstellen belästigt wird. Dadurch, daß eine Unterpolsterung lediglich im Bereich der Achselhöhle des Patienten vorgesehen ist, trägt die Bandage unter der Kleidung des Patienten kaum auf und wird somit von Dritten nicht bemerkt. Außerdem sorgt die Unterpolsterung dafür, daß sich der Patient an den druckempfindlichen Stellen in der Achselhöhlengegend nicht wundreibt, was das Wohlbefinden des Patienten fördert.

Eine erfindungsgemäße Ausgestaltung der Erfindung ist dadurch gekennzeichnet, daß die Bänder im Bereich einer Verbindungsstelle V-förmig direkt miteinander verbunden sind und daß die Verbindungselemente im Bereich der Verbindungsstelle an den jeweiligen Bandenden befestigt sind, so daß die Bänder im angelegten Zustand in der Verbindungsstelle kreuzförmig aufeinanderstoßen, d.h. die durch die Bänder eingeleiteten Zugkräfte sind auf den gleichen Punkt innerhalb der Verbindungsstelle gerichtet. Dadurch wird der gute Sitz im angelegten Zustand der Fertigbandage weiter verbessert.

In einer weiteren Ausgestaltung der Erfindung sind die Verbindungselemente schnallenförmig ausgebildet und weisen einen viereckigen Umriß auf, so daß ein Verrutschen der in die Verbindungselemente eingreifenden freien Bandenden sicher vermieden wird.

Des weiteren ist vorgesehen, daß das Rückenpolster unterhalb der Verbindungselemente mit seitlich herausragenden und auf der Unterseite gepolsterten Verlängerungen versehen ist. Die Unterlegung der Verbindungsstelle der Bandage im Rücken des Patienten mit einem Rückenpolster sorgt dafür, daß der Patient nicht durch Druckstellen belästigt wird.

In Fortführung der Erfindung besteht die Unterseite des Rückenpolsters aus einem aus hautfreundlichen Naturfasern gefertigten Gewebe, wohingegen die Oberseite aus einem Polyamidgewebe besteht, so daß ein äußerst zugfester Verbund geschaffen wird. Dadurch, daß die Seite sowohl der Bänder als auch des Rückenpolsters, die unmittelbar mit der Haut des Patienten in Berührung kommt, aus einem aus Naturfasern bestehenden Gewebe gefertigt ist, werden Reizungen der Haut vermieden und die Gefahr des Auftretens von Allergien verringert sich wesentlich.

Nach einer vorteilhaften Ausführungsform der Erfindung sind die Bänder im Bereich der V-förmigen Verbindungsstelle und der Verbindungselemente mit dem Rückenpolster und/oder miteinander vernäht.

Damit wird verhindert, daß zusätzliche Verbindungselemente zum Verknüpfen der Bänder eingesetzt werden müssen. Außerdem kann dadurch das Rückenpolster nicht verrutschen und erleichtert somit das Anlegen der Bandage.

Nach einer weiteren Ausführung der erfindungsgemäßen Lösung sind die flachen Verbindungselemente im Bereich der V-förmigen Verbindungsstelle an der äußeren Bandlängsseite mit dem jeweiligen Band verbunden und/oder am Rückenpolster befestigt.

Dabei hat es sich als besonders vorteilhaft erwiesen, wenn die Befestigung der Verbindungselemente im Bereich der Bandenden mittels Schlaufen geschieht, deren Enden mit dem jeweiligen Band und/oder dem Rückenpolster vernäht sind.

Dadurch soll gewährleistet werden, daß die Bandage, die über einen verhältnismäßig langen Zeitraum hinweg einen straffen Sitz aufweisen muß, an ihren Verbindungsstellen nicht einreißt und damit unbrauchbar wird. Das Vernähen mit dem Rückenpolster gibt den Bändern und dem Verbindungselement zusätzliche Festigkeit.

Nach einer anderen bevorzugten Ausführungsform ist die Unterpolsterung körperseitig auf den Bändern befestigt und besteht vorzugsweise aus einem Schaumstoffstreifen mit einem doppelten Bezug aus einem aus hautfreundlichen Naturfasern bestehenden Gewebe.

Diese Ausführungsform verhindert das Auftragen der Bandage unter der Kleidung des Patienten, der doppelte Bezug aus einem aus hautfreundlichen Naturfasern bestehenden Gewebe sorgt dafür, daß im Achselhöhlenbereich der Schweiß aufgenommen werden kann und ein Wundreiben an den druckempfindlichen Stellen in der Achselhöhlengegend vermieden wird.

Im unmittelbaren Endbereich der freien Enden der Bänder auf der dem Körper abgewandten Seite sind nach einer weiteren Ausführung der Fertigbandage Verschlußelemente in Form von Klettverschlußelementen angeordnet, wobei die Länge der Klettverschlußelemente dann unabhängig von der Einstellbarkeit der Länge der Bänder ist, wenn, wie vorgesehen, die Oberfläche der dem Körper abgewandten Bandfläche aus einem schlingenartigen Polyamidgewebe besteht.

Klettverschlußelemente sichern einen schnellen, einfachen und festen Verschluß der Bänder im Rücken des Patienten. Sie tragen weder auf, noch verursachen sie Druckstellen. Die Lösbarkeit des Verschlusses geschieht gleichermaßen einfach und schnell. Klettverschlüsse sind sowohl für den Dauerverschluß über einen langen Zeitraum hinweg als auch für kurzzeitige, ständige Öffnungs-und Schließprozesse verwendbar.

Das für die Bänder und das Rückenpolster verwendete Material sichert einerseits, daß die Haut des Patienten nur mit hautfreundlichen Materialien in Berührung kommt. Dazu ist die körperseitige Bandseite aus einem aus Naturfasern bestehenden Gewebe gefertigt. Die dem Körper abgewandte Seite der Bänder und des Rückenpolsters besteht aus einem schlingenbildenden Polyamidmaterial, womit andererseits gewährleistet wird, daß die Fertigbandage undehnbar ist und damit den andauernden straffen Sitz sichert und daß der Klettverschluß in seiner Länge so kurz wie möglich gehalten werden kann, wobei dennoch die Fertigbandage längenvariabel bleibt.

Damit gestaltet sich insgesamt gesehen die Fertigbandage kostengünstig, sie ist unkompliziert zu handhaben, aus waschbaren Materialien hergestellt und damit mehrmals verwendbar. Sie kann ohne großen bzw. zusätzlichen Aufwand in unterschiedlichen Größen gefertigt und den Körpermaßen des Patienten angepaßt werden.

Die Erfindung soll nachfolgend anhand eines Ausführungsbeispieles näher erläutert werden. Die dazugehörige Zeichnung zeigt in
- Fig.1: die Rückenansicht der Fertigbandage im angelegten Zustand und
- Fig. 2: eines der Bänder im Längsschnitt im Bereich der Unterpolsterung.

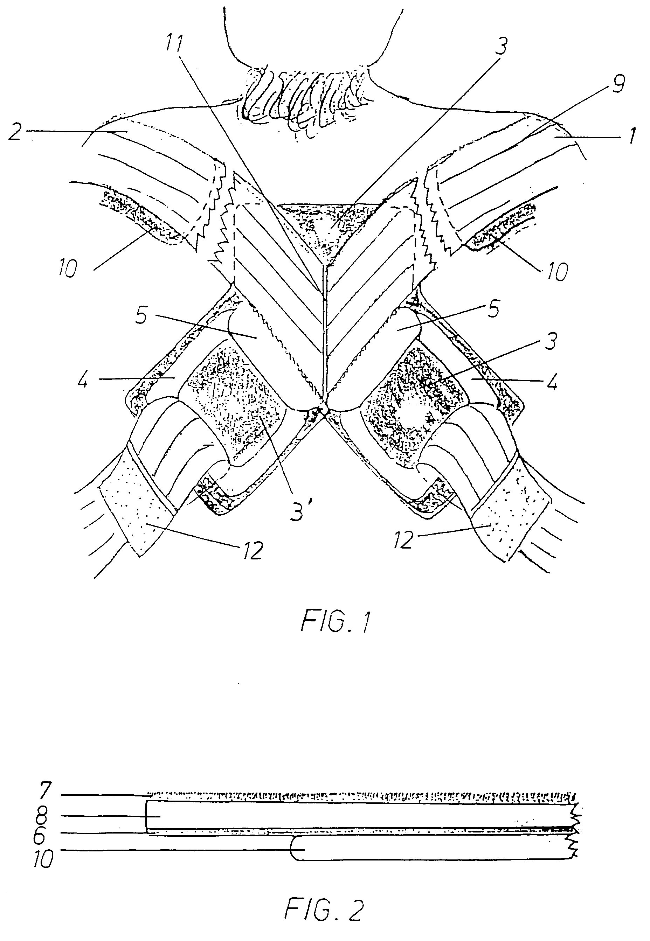

Die Fertigbandage nach Fig. 1 besteht im wesentlichen aus zwei breiten Bändern 1, 2, wobei jeweils ein Ende eines Bandes 1, 2 mit einem anderen Ende des jeweils anderen Bandes 1, 2 an einer Schmalseite V-förmig miteinander verbunden und gleichzeitig auf ein Rückenpolster 3 aufgenäht wird. Unterhalb dieser Verbindungsstelle sind seitlich an den miteinander verbundenen Enden der Bänder 1, 2 zwei Verbindungselemente 4 mittels Befestigungsschlaufen 5 mit den Bändern 1, 2 verbunden. Damit können die freien Enden der Fertigbandage durch jeweils ein Verbindungselement 4 gezogen und schnallenartig befestigt werden.

Die Bänder 1, 2 und die Rückenpolster 3 sind, wie aus Fig. 2 ersichtlich, aus unterschiedlichen Materialschichten gefertigt. Die Unterseiten 6 der Bänder 1, 2 und die Unterseite des Rückenpolsters 3, die unmittelbar mit der Haut des Patienten in Berührung kommen, bestehen aus einem hautfreundlichen Textilgewebe aus Naturfasern und schützen so vor Schweiß, Juckreiz und Allergien. Um zu gewährleisten, daß die Fertigbandage über lange Zeit hinweg einen straffen, unveränderlichen Sitz aufweist, darf sich die Bandage möglichst nicht dehnen. Deshalb sind die dem Körper abgewandten Oberseiten 7 der Bänder 1, 2 und auch die Oberseite des Rückenpolsters 3 aus einem nahezu undehnbaren Material, wie zum Beispiel aus einem Schlaufen bildenden Polyamid/Nylonvelourgewebe hergestellt.

Bevor beide Materialien übereinanderliegend vernäht werden, wird eine dünne Polsterschicht 8 aus Schaumstoff zwischen beide Bandseiten 6, 7 und auch zwischen Ober- und Unterseite des Rückenpolsters 3 eingebracht. Damit wird der Tragekomfort der Bandage erhöht. Zur zusätzlichen Fixierung und zum besseren Sitz (kein Verrutschen) der Bandseiten 6, 7 und der Polsterschicht 8 sind Steppnähte 9, die in Bandlängsrichtung verlaufen, vorgesehen.

Auf die Unterseite 6 der Bänder 1, 2 sind im Bereich der Achselhöhlen Unterpolsterungen 10 aufgenäht, die aus einem etwa 15 mm dicken Schaumstoffstreifen bestehen und mit einem doppelten Überzug aus reiner Baumwolle versehen sind. Damit wird erreicht, daß die Bandage auf den druckempfindlichen Hautpartien nicht zum Wundreiben führt. Der Tragekomfort wird dadurch noch verbessert, daß der doppelte Baumwollüberzug den Körperschweiß im Achselhöhlenbereich aufnehmen kann.

Dadurch, daß nur an den Stellen der Bänder 1, 2 dicke Unterpolsterungen 10 vorgesehen sind, an denen sie unbedingt notwendig sind, nämlich im Bereich der wirklich empfindlichen Achselhöhlen, wird verhindert, daß die Fertigbandage unter der Kleidung aufträgt und den Patienten verunsichert.

Das Rückenpolster 3 weist im wesentlichen eine solche Form und Größe auf, daß mindestens die beiden Bandenden, die mit je einer ihrer Schmalseiten V-förmig aneinandergenäht sind, unterlegt sind. Des weiteren befinden sich an dem Rückenpolster auf der Unterseite ebenfalls mit einer Polsterung versehene Verlängerungen 3', die sich unterhalb der im Bereich der V-förmigen Verbindungsstelle 11 beider Bandenden befestigten Verbindungselemente 4 erstrecken. Damit wird einerseits gesichert, daß das Rückenpolster 3 seine vorbestimmte Lage behält und nicht verrutschen kann und andererseits erreicht, daß die Nahtstellen zwischen den Bändern 1, 2 und den Befestigungsschlaufen 5, die ständig unter Zugbelastung stehen, eine zusätzliche Festigkeit erhalten und so die Lebensdauer der Fertigbandage erhöht wird.

Die beiden Verbindungselemente 4 sind im Ausführungsbeispiel entsprechend Fig. 1 als flache, viereckige, individuell einstellbare Kunststoffschnallen ausgebildet, durch die die freien Enden der Bänder 1, 2 nach dem Anlegen der Bandage hindurchgeführt sind. Mittels auf der Oberseite 7 der Bänder 1, 2 im unmittelbaren Endbereich befestigten Klettverschlußelemente 12 werden je nach Längenbedarf und Sitz der Bandage, die Klettverschlußelemente 12 mit der Oberseite 7 der Bänder 1, 2 verbunden. Die Klettverschlußelemente 12, die vorteilhaft als einzelne Klettstreifen ausgebildet sind, können dabei in ihrer Länge so klein wie möglich gehalten werden, da sie als Gegenelemente keine weiteren Klettstreifen benötigen, sondern auf dem schlaufenbildenden Polyamidmaterial gut und dauerhaft haften. Damit sind die Bänder 1, 2 auf einfache Art und Weise in ihrer Länge variabel einstellbar.

Das Anlegen der Bandage geschieht rucksackartig dadurch, daß die V-förmige Verbindungsstelle 11 der Bänder 1, 2 mit dem Rückenpolster 3 auf den Rücken des Patienten etwa in Schulterhöhe aufgelegt, die Bänder 1, 2 über die rechte beziehungsweise linke Schulter auf die Körpervorderseite und durch die Achselhöhlen wieder zum Rücken geführt werden, dort die Bandenden mit den Klettverschlußelementen 12 durch das jeweilige Verbindungselement 4 hindurchgezogen und die Bandage mittels der Klettverschlußelemente 12 straff verschlossen wird.

Die erfindungsgemäße Fertigbandage ist waschbar, schnell und unkompliziert anleg- und entfernbar und damit kostengünstig und zeitsparend. Durch den Einsatz unterschiedlicher Gewebe für Ober- und Unterseite 6,7 der Bänder 1,2 und des Rückenpolsters 3 begünstigt sie das Wohlbefinden des Patienten und besitzt gute Trageeigenschaften. Der Heilungsprozeß wird dadurch positiv beeinflußt, daß die Fertigbandage einen anatomisch guten und festen Sitz über einen langen Zeitraum garantiert. Die Fertigbandage kann weder verrutschen noch sich lockern, ein Nachspannen ist nicht erforderlich. Ohne weiteren, größeren Aufwand ist es leicht möglich, die Bandage in unterschiedlichen Größen, die den üblichen Konfektiongrößen angepaßt sein können, herzustellen.

### Bezugszeichenliste

- 1: Band
- 2: Band
- 3: Rückenpolster
- 3': Verlängerung
- 4: Verbindungselement
- 5: Befestigungsschlaufe
- 6: Unterseite
- 7: Oberseite
- 8: Polsterschicht
- 9: Steppnähte
- 10: Unterpolsterung
- 11: Verbindungsstelle
- 12: Klettverschlußelement

## Patentansprüche

1. Fertigbandage insbesondere zur Behandlung von Schlüsselbeinverletzungen, die im wesentlichen aus zwei in ihrer Länge einstellbaren Bändern aus einem nicht dehnbaren Material besteht, wobei die Bänder jeweils an einer ihrer Schmalseiten flachliegend miteinander verbunden und in einem festen Winkel zueinander angeordnet sind, im Bereich der Verbindung beider Bänder mindestens ein flaches Verbindungselement befestigt ist und die Bandage dem Patienten derart angelegt wird, daß die Bänder jeweils von der Schulter des Patienten direkt zum Rücken und von der Schulter durch die Achselhöhlen des Patienten zum Rücken verlaufen und dort miteinander verbunden sind, **dadurch gekennzeichnet,** daß die Bänder (1, 2) über die gesamte Länge und Breite dreischichtig ausgebildet sind, derart, daß die dem Körper des Patienten zugewandte Unterseite (6) aus einer naturfasernen Textilgewebeschicht besteht, auf der eine dünne Polsterschicht (8) aus einem Schaumstoffmaterial und darauf als Oberseite (7) eine Schicht aus einem Polyamidgewebe angeordnet ist und die drei Schichten (6, 7, 8) über ihre gesamte Länge miteinander fest verbunden sind, daß an der Unterseite (6) im Bereich der direkten Verbindung beider Bänder (1, 2) miteinander ein Rückenpolster (3, 3') angeordnet ist und daß die Bänder (1, 2) lediglich im Bereich der Achselhöhlen mit einer zusätzlichen Unterpolsterung (10) versehen sind.

2. Fertigbandage nach Anspruch 1, **dadurch gekennzeichnet,** daß die Bänder (1, 2) im Bereich einer Verbindungsstelle (11) V-förmig direkt miteinander verbunden sind und daß die Verbindungselemente (4) im Bereich der Verbindungsstelle (11) an den jeweiligen Bandenden befestigt sind, so daß die Bänder (1, 2) im angelegten Zustand in der Verbindungsstelle (11) kreuzförmig aufeinanderstoßen.

3. Fertigbandage nach Anspruch 1 und 2, **dadurch gekennzeichnet,** daß die Verbindungselemente (4) schnallenförmig ausgebildet sind und einen viereckigen Umriß aufweisen.

4. Fertigbandage nach den Ansprüchen 1 bis 3, **dadurch gekennzeichnet,** daß das Rückenpolster (3) unterhalb der Verbindungselemente (4) mit seitlich herausragenden und auf der Unterseite gepolsterten Verlängerungen (3') versehen ist.

5. Fertigbandage nach den Ansprüchen 1 bis 4, **dadurch gekennzeichnet,** daß die Unterseite des Rückenpolsters (3) aus einem aus hautfreundlichen Naturfasern bestehenden Gewebe und die Oberseite des Rückenpolsters (3) aus einem Polyamidgewebe besteht.

6. Fertigbandage nach Anspruch 1 bis 5, **dadurch gekennzeichnet**, daß die Bänder (1, 2) im Bereich der Verbindungsstelle (11) und der Verbindungselemente (4) mit dem Rückenpolster (3) und/oder miteinander vernäht sind.

7. Fertigbandage nach Anspruch 1 bis 6, **dadurch gekennzeichnet,** daß die flachen Verbindungselemente (4) im Bereich der Verbindungsstelle (11) an der äußeren Bandlängsseite mit dem jeweiligen Band (1, 2) verbunden und/oder am Rückenpolster (3) befestigt sind.

8. Fertigbandage nach Anspruch 7, **dadurch gekennzeichnet,** daß die Befestigung der Verbindungselemente (4) im Bereich der Bandenden mittels Befestigungsschlaufen (5) geschieht, deren Enden mit dem jeweiligen Band (1, 2) und/oder dem Rückenpolster (3) vernäht sind.

9. Fertigbandage nach Anspruch 1 bis 8, **dadurch gekennzeichnet,** daß die Unterpolsterung (10) auf der Unterseite (6) der Bänder (1, 2) befestigt ist und vorzugsweise aus einem Schaumstoffstreifen mit einem doppelten Bezug aus einem aus hautfreundlichen Naturfasern hergestellten Gewebe besteht.

10. Fertigbandage nach Anspruch 1 bis 9, **dadurch gekennzeichnet,** daß die freien Enden der Bänder (1, 2) auf der Unterseite (6) im unmittelbaren Endbereich Verschlußelemente in Form von Klettverschlußelementen (12) aufweisen.

11. Fertigbandage nach Anspruch 10, **dadurch gekennzeichnet,** daß die Länge der Klettverschlußelemente (12) unabhängig von der Einstellbarkeit der Länge der Bänder (1, 2) ist.

12. Fertigbandage nach Anspruch 1 bis 11, **dadurch gekennzeichnet,** daß die Oberfläche der dem Körper abgewandten Bandfläche aus einem schlingenartigen Polyamid-Gewebe besteht.
